(19) Europäisches Patentamt
European Patent Office
Office européen des brevets

(11) EP 0 701 815 B1

(12) EUROPÄISCHE PATENTSCHRIFT

(45) Veröffentlichungstag und Bekanntmachung des Hinweises auf die Patenterteilung:
30.06.1999 Patentblatt 1999/26

(51) Int. Cl.$^6$: A61K 9/14, A61K 9/16

(21) Anmeldenummer: 95113398.2

(22) Anmeldetag: 18.01.1993

(54) **Verfahren zur Herstellung von Wirkstoff enthaltenden Pulvern, Granulaten oder Pellets mit einem Gerüst aus hydrophilen Makromolekülen und ihre Verwendung**

Process for producing powders, granulates or pellets containing active substances and a structure consisting of hydrophilic macromolecules, and use thereof

Procédé de préparation de poudres, granules ou pellets renfermant une substance active et ayant une structure composée de macromolécules hydrophiles, ainsi que leur utilisation

(84) Benannte Vertragsstaaten:
AT BE CH DE DK ES FR GB GR IT LI NL PT SE

(30) Priorität: 17.01.1992 DE 4201179
17.01.1992 DE 4201173
30.04.1992 US 876864
30.04.1992 US 876877

(43) Veröffentlichungstag der Anmeldung:
20.03.1996 Patentblatt 1996/12

(62) Dokumentnummer(n) der früheren Anmeldung(en) nach Art. 76 EPÜ:
93902034.3 / 0 621 777

(73) Patentinhaber:
ALFATEC-PHARMA GmbH
69120 Heidelberg (DE)

(72) Erfinder:
• Wunderlich, Jens-Christian
D-69126 Heidelberg (DE)
• Schick, Ursula
D-69198 Schriesheim (DE)
• Werry, Jürgen
D-67071 Ludwigshafen (DE)
• Freidenreich, Jürgen
D-69198 Schriesheim (DE)

(74) Vertreter:
Winter, Brandl, Fürniss, Hübner, Röss, Kaiser, Polte, Kindermann
Partnerschaft
Patent- und Rechtsanwaltskanzlei
Alois-Steinecker-Strasse 22
85354 Freising (DE)

(56) Entgegenhaltungen:
EP-A- 0 081 912          EP-A- 0 138 216
WO-A-88/04551          WO-A-91/09591
WO-A-93/13761          WO-A-94/01090
US-A- 4 837 285

• CHEMICAL ABSTRACTS, vol. 113, no. 13, 24.September 1990 Columbus, Ohio, US; abstract no. 114127d, 'MANUFACTURE OF GRANULATED FOODS CONTAINING OILS AND SACCHARIDES' & JP-A-02 135 052 (TAIYO CHEMICAL) 23.Mai 1990
• JOURNAL OF MICROENCAPSULATION, Bd. 7, Nr. 2, 1990 Seiten 209-217, S.E. LEUCUTA 'CONTROLLED RELEASE OF NIFEDIPINE FROM GELATIN MICROSPHERES AND MICROCAPSULES'

**Beschreibung**

Die Erfindung betrifft ein neues Verfahren zur Herstellung von exakt dosierbaren Pulvern, Granulaten oder Pellets aus hydropilen Makromolekülen, Wirkstoffen und gegebenenfalls weiteren pharmazeutisch akzeptablen Gerüst- und Hilfsstoffen, wobei der Wirkstoff in einer Matrix gelöst oder suspendiert vorliegt, sowie weiterhin ihre Verwendung als Arzneimittel, Kosmetikum, Diagnostikum oder diätetisches Lebensmittel (health care).

WO-A-9109591 (Mediventures) offenbart ein Verfahren zur Herstellung von pharmazeutischen und anderen Matrixsystemen, bei denen man eine in einem ersten Lösungsmittel gelöste oder dispergierte Matrixverbindung verfestigt und anschließend die verfestigte Matrix mit einem zweiten Lösungsmittel, das im wesentlichen mit dem ersten Lösungsmittel bei einer niedrigeren Temperatur als dem Gefrierpunkt des ersten Lösungsmittels mischbar ist, behandelt. Die Matrixverbindung ist im wesentlichen in dem zweiten Lösuungsmittel unlöslich. Hierdurch wird das erste Lösungsmittel im wesentlichen entfernt, was zu einer brauchbaren Matrix führt (Lösungsmittelaustauch).

Granulate bzw. Pellets als Formkörper dienen in der pharmazeutischen Industrie hauptsächlich als Zwischenprodukte zur Tablettierung. Die Formgebung soll dabei zu einem frei fließenden, körnigen und staubfreien Produkt führen, das aufgrund seiner Gleichförmigkeit die technologische Verarbeitung und die Dosiergenauigkeit verbessert. Darüberhinaus besitzen Pellets als moderne multiple-unit-Arzneiform, beispielsweise abgefüllt in Hartgelatinekapseln, gegenüber single-unit-Arzneiformen, wie z.B. Tabletten oder Dragees, eine Reihe von Vorteilen:

- Sie verteilen sich gleichmäßig im Gastrointestinaltrakt.
- Aufgrund ihrer geringen Größe resultieren im Gegensatz zu monolithischen Arzneiformen kürzere Magenverweilzeiten, vor allem bei magensaftresistent überzogenen Arzneiformen.
- Sie lösen sich im Gastrointestinaltrakt als Einzelaggregate im Gegensatz zu einer komprimierten Tablette, die erst in ihre Granulatteilchen zerfallen muß, schneller auf.
- Es können Pellets mit unterschiedlicher Wirkstoffabgabe in gemischter Form einzeldosiert werden.

Jedoch liegt allen Verfahren des Standes der Technik die grundsätzliche Problematik der notwendigen Formgebung von pulvrig-kristallinen Wirk- und Hilfsstoffen zu verarbeitbaren Granulaten (Pellets) als Formkörper zugrunde.

Man unterscheidet dabei zwischen auf- und abbauenden Verfahren. Allen Verfahren gemeinsam ist, daß man bislang nur über mehrere und aufwendige Teilschritte zu Granulaten bzw. Pellets als Formkörper gelangt.

Bei den abbauenden Verfahren werden - vereinfacht dargestellt - zunächst die Arznei- und Hilfsstoffe zerkleinert, durch Sieben auf eine einheitliche Korngröße gebracht und dann gemischt. Danach erfolgt das trockene oder feuchte Granulieren, bei dem die Pulvermischung aggregiert und anschließend zu Granulatkörnern zerkleinert wird. Im nächsten Schritt wird, wenn nötig, getrocknet und wiederum gesiebt.

Bei den Aufbaugranulaten werden aus den pulverförmigen Arznei- und Hilfsstoffen unter kontinuierlicher Zugabe von Granulierflüssigkeit bei gleichzeitigem Trocknen Granulatkörner in einem kontrollierten Prozeß (z.B. Wirbelschichtverfahren) gebildet.

Durch anschließende, spezielle Ausrundungsverfahren (z.B. Marumerizer[R]) gelangt man zu runden, kugelförmigen Granulatteilchen (Pellets). Nachteilig hierbei ist, daß bei der Ausrundung von bereits hergestellten, unförmigen Granulatteilchen Substanzmasse mit Arzneistoff verlorengeht und nicht direkt dem Granulierprozeß wieder zugeführt werden kann. Dies stellt sicher ein kosten - und entsorgungstechnisches Problem dar. Gleichzeitig führt die mechanische Ausformung zu einem ungleichförmigen Produkt.

Spezielle Pelletiertechniken sind beispielsweise die aufbauende Trockenpelletierung durch Kompaktierung und die Wirbelschichtgranulierung, die sehr unbefriedigende Ergebnisse hinsichtlich Form und mechanischer Festigkeit der Pellets liefern.

Alle diese Herstellungsprozesse stellen technologisch aufwendige Mehrschrittverfahren dar. Sie sind gekennzeichnet durch eine Vielzahl von Prozeßparametern technologischer Art, wie z.B. Temperatur, Feuchtigkeitsgehalt, Homogenität der Mischungen u.s.w..

Weiterhin ist bei allen Granulations- und Pelletierverfahren der Einsatz einer ganzen Reihe von Hilfsstoffen notwendig. So müssen beispielsweise Bindemittel oder Granulierflüssigkeiten eingesetzt werden, um das pulverförmige Gut in eine feste, kompakte und verarbeitungsfähige Form zu bringen. Genaueste Kenntnisse um das physikalisch-chemische Verhalten z.B. Lösungswärme, Löslichkeit oder Kristallbildungstendenz und große Erfahrung im Umgang mit diesen Stoffen ist notwendig, um das Zusammenwirken dieser Hilfsstoffe untereinander und mit dem Arzneistoff im Zusammenhang mit allen zu beachtenden Prozeßparametern beurteilen zu können.

Somit können die pharmazeutischen Anforderungen an ein Granulat (Pellets) oft nur durch empirische Versuche in Abhängigkeit des zur Verarbeitung kommenden Arzneistoffs und der daraus zu formulierenden Darreichungsform erfüllt werden.

Daher wird verständlich, daß die Einhaltung konstanter Herstellungsbedingungen bei den aufwendigen Verfahren sehr schwierig ist. So ist es, bedingt durch die Vielzahl von zu beachtenden Parametern, bei den bekannten Herstellungsprozessen trotz eines hohen Entwicklungs- und Optimierungsaufwandes nicht für jeden Arzneistoff möglich, ein geeignetes Verfahren zu finden.

Betrachtet man nach dem Stand der Technik hergestellte Pellets oder Granulate darüberhinaus unter biopharmazeutischen Aspekten, so läßt sich erkennen, daß der Arzneistoff aus diesen aggregierten Formkörpern erst nach Desaggregation und anschließende Freisetzung dem Organismus zur Verfügung gestellt werden kann. Die Vielzahl prinzipiell unterschiedlicher Haft- und Bindungskräfte in Granulaten verdeutlicht diese Problematik. Durch erhärtende Bindemittel beim Trocknen (Feuchtgranulierung) oder durch Sintern bzw. Schmelzhaftung unter Druckeinwirkung (Trockengranulierung) entstehen Feststoffbrücken, deren bindende Kräfte im Organismus überwunden werden müssen, um den Arzneistoff überhaupt erst aus der Arzneiform freizugeben.

Jeder Herstellungsschritt bei den Verfahren des Standes der Technik kann somit einen ungünstigen Einfluß auf die Freisetzung des Wirkstoffs und damit auf seine Bioverfügbarkeit nehmen.

Die vorliegende Erfindung stellt sich die Aufgabe, ein Verfahren zur Herstellung neuartiger Festkörper sowie Mischungen vorzuschlagen, die zum einen aufgrund ihrer Struktur und Zusammensetzung die Bioverfügbarkeit und Verträglichkeit von Arzneistoffen verbessern: lagerstabil, exakt dosierbar, als single oder multiple unit vorliegen und zum anderen auf umweltschonende, einfache und wirtschaftliche Weise herzustellen sind, die Wirkstoffe auf schonende Weise verarbeiten und somit insgesamt gesehen die Nachteile des Standes der Technik überwinden.

Diese Aufgabe wird erfindungsgemäß durch ein Verfahren zur Herstellung von wenigstens einen nun wasserlöslichen Wirkstoff enthaltenden Pulvern, Granulaten oder Pellets gelöst, daß dadurch gekennzeichnet ist, daß man

a) einen Gerüstbildner aus hydrophilen Makromolekülen ausgewählt aus der Gruppe bestehend aus: Kollagen, Gelatine, fraktionierte Gelatine, Kollagenhydrolysate, Gelatinederivate, Pflanzenproteine, Pflanzenproeinhydrolysate, Elastinhydrolysate, in einem wäßrigen oder wäßrig-organischen Lösungsmittel löst,

b) den Wirkstoff in der Lösung gemäß a) löst oder suspendiert und

c) die erhaltene Mischung aus gelöstem Gerüstbildner und gelöstem oder suspendiertem Wirkstoff in ein tiefkaltes inertes verflüssigtes Gas eintropft und so Pulver, Granulate oder Pellets formt, und

d) die so geformten Pulver, Granulate oder Pellets durch Verdampfen oder Sublimieren des Lösungsmittels auf übliche Weise trocknet.

Unter Festkörper wird im Sinne der Erfindung ein solcher verstanden, der ausgwählt ist aus der Gruppe bestehend aus: Pulver, Granulate, Pellets, Mikropellets im wesentlichen symmetrisch ausgebildete Aggregate.

Erfindungsgemäß sind einheitlich runde Festkörper, insbesondere Pellets, besonders für pharmazeutische Anwendungen geeignet, wobei der Begriff Pellet vorzugsweise einen Korngrößenbereich von etwa 0,2 bis 12 mm umfaßt.

In der Beschreibung der Erfindung werden die Eigenschaften, Herstellung und Verwendung anhand von runden Pellets bevorzugt dargestellt.

Jedoch kann der Fachmann auch andere Festkörper aus der Gruppe bestehend aus: Pulver, Granulate, im wesentlichen symmetrisch ausgebildete Aggregate, vorteilhaft zur Herstellung, insbesondere von Arzneiformen, einsetzen.

Bevorzugte Ausführungsformen der Erfindung sind in den Unteransprüchen beschrieben und beansprucht.

Die erfindungsgemäß hergestellten festen bis halbfesten oder gelförmigen Pellets stellen runde, einheitliche Formkörper im Bereich von 0,2 - 12 mm dar. Pellets im Bereich von 0,2 - 2 mm eignen sich für multiple unit dosage forms, Pellets im Bereich von 2 - 12 mm sind als single unit dosage-Form verwendbar.

Im Hinblick auf die Arzneimittelsicherheit wird von der pharmazeutischen Industrie exakte Dosiergenauigkeit, Homogenität, Verträglichkeit und Lagerstabilität der entsprechenden Arzneiform gefordert. Bei herkömmlicher Arzneimittelherstellung ist dieser Standard oft nur mit hohem und kostenintensivem Aufwand zu erreichen. Die einheitliche Korngrößenverteilung der beanspruchten Pellets, verbunden mit einer homogenen Verteilung des Arzneistoffes, verbessern gegenüber dem Stand der Technik die Dosiergenauigkeit deutlich. Weiterhin werden die in der Pelletmatrix eingebetteten Wirkstoffe in eine lagerstabile Form gebracht, die eine hohe mechanische Festigkeit mit geringem Abrieb (Friabilität) aufweist. Empfindliche Wirkstoffe werden zudem vor äußeren Einflüssen zuverlässig schützt.

Die erfindungsgemäß hergestellten Pellets als Formkörper, die sich durch ihre einheitliche runde und gleichmäßige Gestalt auszeichnen, sind aufgrund ihres harmonischen Gesamteindruckes optisch sehr ansprechend und können die Akzeptanz beim Patienten steigern. Durch entsprechende Farbgebung lassen sich die klar durchsichtigen und glänzenden, opak bis transparent oder updurchsichtig aussehenden Pellets zu unverwechselbaren Arzneispezialitäten entwikkeln.

Durch die vorteilhafte Schutzkolloidfunktion der beanspruchten Makromoleküle und die gleichzeitige Einbettung der Wirkstoffe in dem polymeren Matrixgerüst wird insbesondere bei schleimhautirritierenden Wirkstoffen die Verträglichkeit deutlich erhöht. So kann z.B. die Magenschleimhautreizung bzw. Irritation von Acetylsalicylsäure durch die schleimhautprotektive Wirkung der beanspruchten Makromoleküle wirkungsvoll vermindert werden (vergl. Beispiel 7) Als Arzneiform sind die beschriebenen Pellets wohlschmeckend und peroral gut einzunehmen.

Überraschenderweise erfolgt bei allen Arzneistoffen unabhängig davon, ob sie gelöst oder suspendiert in den erfindungsgemäßen Pellets als Formkörper vorliegen, im Gegensatz zu herkömmlichen Granulaten, Pellets oder Tabletten die Freisetzung des aktiven Agens im Organismus ohne vorgelagerten Zerfallsprozeß. Bei herkömmlichen Zubereitungen müssen zunächst die Haft- und Bindekräfte, die eine Formgebung überhaupt ermöglichen, überwunden werden, desweiteren müssen die so erhaltenen Unteraggregate benetzt und gelöst werden, bis der Arzneistoff schließlich in einer resorptionsfähigen Form vorliegt. Herkömmliche feste Arzneiformen können je nach Art der verwendeten Hilfsstoffe und dem angewandten Herstellungsverfahren die Bioverfügbarkeit von Wirkstoffen erheblich herabsetzen.

Der Lösevorgang aus der Arzneiform als zeitbestimmender Faktor hängt bei den erfindungsgemäßen Pellets als Formkörper ausschließlich von der Art und Zusammensetzung des hydrophilen Matrixsystems ab und ist in der Freisetzungsrate modulierbar. So können Akutformen, die sich innerhalb von wenigen Sekunden auflösen, als auch Retardformen formuliert werden. Die Auflösung des Gerüstbildnders ist der geschwindigkeitsbestimmende Schritt.

Offensichtlich führt also das Vorliegen in einer erfindungsgemäß hergestellten Zubereitung zu einer stark erhöhten (effektiveren) Resorption der Arzneistoffdosis unter physiologischen Bedingungen.

Die arzneistoffhaltigen Pellets sind während des schonenden Herstellungsprozesses (Formgebung) niedrigen Temperaturen ausgesetzt und kommen nur mit einem inerten Medium (flüssiger Stickstoff) in Berührung. Daher erfolgt eine Veränderung der Arzneisstoffe oder eine Kontamination mit Rückständen von Kühlölen bzw. organischen Lösungsmitteln, wie beispielsweise von der klassischen Weichgelatinekapselherstellung bekannt ist, nicht.

Unter technologischen und biopharmazeutischen Aspekten erfüllen die beschriebenen Pellets alle prinzipiellen Anforderungen, die an diese Dosierungsform zu stellen sind:

- Sie sind in Form und Farbe gleichmäßig,
- besitzen eine enge Korngrößenverteilung,
- sind leicht dosier-und abfüllbar,
- weisen eine hohe mechanische Festigkeit und Haltbarkeit auf,
- setzen den Arzneistoff schnell oder moduliert frei.

Im Sinne der Erfindung können - allein oder in Mischungen - hydrophile Makromoleküle aus der Gruppe bestehend aus: Kollagen, Gelatine, fraktionierte Gelatine, Gelatinederivate, Kollagenhydrolysate, Pflanzenproteine Pflanzenproteinhydrolysate, Elastinhydrolysate, eingesetzt werden.

Diese biogenen Stoffe sind pharmazeutisch unbedenklich und untoxisch. Die Matrixeigenschaften der genannten Eiweißstoffe lassen sich in genauer Kenntnis ihres chemisch-physikalischen Verhaltens in weiten Grenzen einstellen und führen so zu einem Arzneimittel, bei dem der jeweilige Wirkstoff in optimaler und reproduzierbarer Form vorliegt.

Gelatine ist ein aus kollagenhaltigem Material gewonnenes Skleroprotein, das je nach Herstellungsprozeß unterschiedliche Eigenschaften hat. Sie besteht im wesentlichen aus vier Molekulargewichtsfraktionen, die die physikalisch-chemischen Eigenschaften in Abhängigkeit vom Molekulargewicht und prozentualem Gewichtsanteil beeinflussen. Je höher z.B. der Anteil Mikrogel ($10^7$ bis $10^8$ D) liegt, desto höher ist auch die Viskosität der wäßrigen Lösung. Handelsübliche Sorten enthalten bis zu 10 Gewichtsprozent. Die Fraktionen der alpha-Gelatine und deren Oligomere ($9,5 \times 10^4$ / $10^5$ bis $10^6$ D) sind entscheidend für die Gelfestigkeit und liegen üblicherweise zwischen 10 und 40 Gewichtsprozent. Molekulargewichte unterhalb der alpha-Gelatine werden als Peptide bezeichnet und können in herkömmlichen Gelatinequalitäten (niedrigbloomig) bis zu 80 Gewichtsprozent betragen.

Gelatine besitzt ein temperatur- und konzentrationsabhängiges reversibles Sol-Gel-Umwandlungsverhalten, das von der molekularen Zusammensetzung abhängig ist. Als Maß für das Gelbildungsvermögen der Gelatine ist es international gebräuchlich, die Bloomzahl anzugeben. Niedrige Handelsqualitäten beginnen bei 50 Bloom, hochbloomige Sorten liegen bei etwa 300 Bloom.

Die chemischen und physikalischen Eigenschaften variieren je nach Herstellungsverfahren, wobei besonders schonend gewonnene Gelatinesorten (geringer Anteil an rechtsdrehenden Aminosäuren und Peptiden) kurze Sol-Gel-Umwandlungsgeschwindigkeiten und Schmelzpunkte oberhalb 37°C (gemessen als 10%ige Lösung) aufweisen.

Fraktionierte Gelatine stellt den Spezialfall von Gelatine dar und wird durch spezielle Herstellungstechniken, wie z.B. Ultrafiltration aus herkömmlicher Gelatine gewonnen.

Die Zusammensetzung kann z.B. durch Entfernung von Peptiden (MG < $9,5 \times 10^4$ D) oder durch Mischungen aus Einzelfraktionen wie z.B. alpha-Ketten, dimeren und trimeren Ketten oder Mikrogel variiert werden.

Darüber hinaus hat Gelatine bzw. fraktionierte Gelatine gute Tensideigenschaften mit Schutzkolloidwirkung und Emulgatoreigenschaft.

Kollagen in nativer Form ist wasserunlöslich. Durch spezielle Herstellungsverfahren gibt es heute lösliche Kollagentypen mit einem durchschnittlichen Molekulargewicht von ca. 300.000 D.

Gelatinederivate sind chemisch veränderte Gelatinen, wie z.B. succinylierte Gelatine, die z.B. für Plasmaexpander verwendet werden.

Unter Kollagenhydrolysat wird ein von Kollagen oder Gelatine druckhydrolytisch oder enzymatisch gewonnenes Produkt verstanden, das kein Sol-Gel-Umwandlungsvermögen mehr aufweist. Kollagenhydrolysate sind leicht kaltwasserlöslich und die Molekulargewichtszusammensetzung kann zwischen einigen Hundert D bis unterhalb von $9{,}5 \times 10^4$ D liegen. Auf enzymatischem Wege gewonnene Produkte sind in der molekularen Zusammensetzung homogener und zeigen noch gute Tensid- und Emulgatorwirkung.

Neuentwickelte Produkte stellen die Pflanzenproteine und deren Hydrolysate dar, die in ihren Eigenschaften weitgehend den Kollagenhydrolysaten entsprechen. Sie werden vorzugsweise aus Weizen und Soja gewonnen und besitzen beispielsweise Molekulargewichte von ca. 200.000-300.000 D bzw. ca. 1.000-10.000 D.

Elastinhydrolysate werden enzymatisch aus Elastin gewonnen und bestehen aus einer einzigen Polypeptidkette. Aufgrund ihres hohen Anteils an nichtpolaren Aminosäuren können sie in lipophilen Systemen verwendet werden. Elastinhydrolysate weisen ein Molekulargewicht von ca. 2.000 - 3.000 D auf und sind auf der Haut stark filmbildend.

Bei Verwendung von Pflanzenproteinen, Pflanzenproteinhydrolysaten, Elastinhydrolysaten, bzw. von Kollagenhydrolysaten (kaltwasserlösliche Gelatinen) oder Gelatinen mit einem Maximum der Molekulargewichtsverteilung von einigen Hundert D bis unterhalb von $10^5$ D (Variante A) bildet das Trägermaterial der beanspruchten Formkörper nach einer bevorzugten Ausführungsform der Erfindung durchgeführten Lyophilisation überraschenderweise eine hochporöse und gleichzeitig mechanisch stabile Matrix aus, die sich in kaltem Wasser schnell und vollständig auflöst.

Liegt der Arzneistoff in der Matrix in gelöster oder suspendierter Form vor, sind alle aufgeführten hydrophilen Makromoleküle in den angegebenen Molekulargewichtsbereichen allein oder in Mischungen erfindungsgemäß geeignet.

Besonders geeignet sind enzymatisch gewonnene Hydrolysate, die ein Molekulargewicht zwischen ca. 15.000 und 20.000 D aufweisen.

Die schnelle Auflösung der beschriebenen Matrixrezepturen eignet sich für pharmazeutischen Akutformen, bei denen der Wirkstoff einzeln oder mehrfach dosiert vorliegen kann.

Zur innerlichen Anwendung lassen sich aus den erfindungsgemäßen Pellets als Formkörper vorteilhaft Instantzubereitungen formulieren. Wird z.B. der Wirkstoff in einer schnell auflösenden Matrix eingebettet und pelletiert, so erhält man lagerstabile Pellets, die man (z.B. in Beutel abgefüllt) innerhalb weniger Sekunden in kaltem Wasser vollständig auflösen kann.

Erfindungsgmäß können auch hydrophile Makromoleküle mit sol-gel-bildenden Eigenschaften wie z.B. Gelatine und fraktionierte Gelatine geeignet sein, die ein Maximum der Molekulargewichtsverteilung oberhalb $10^5$ D besitzen, als Gerüstsubstanzen geeignet sein.

Liegt der Arzneistoff in gelöster, oder suspendierter Form in einer sol-gel-bildenden Gerüstmatrix (Variante B) wie Gelatine oder fraktionierte Gelatine vor, so erhält man Pellets, die den Wirkstoff - je nach molekularer Zusammensetzung der verwendeten Gelatinesorte - schnell oder langsam in wäßrigem Medium bei 37°C freisetzen.

In einer weiteren Ausführungsform der Erfindung können Zusätze von Weichmachern von 1-50% (bezogen auf die zu verarbeitende Masse) ausgewählt aus der Gruppe bestehend aus: Glycerol, Propylenglykol, Polyethylenglykole, Triacetin, Sorbitol, Sorbitangemische, Sorbitollösungen, Glucosesirup und andere Polyole bzw. Zuckeralkohole, geeignet sein. Die genannten Stoffe beeinflussen die erfindungsgemäße Matrix hinsichtlich der Konsistenz von fest bis halbfest oder gelförmig, ihr Auflöseverhalten und die Viskosität. Besonders vorteilhaft eignet sich als Weichmacher Sorbitol, der als Süßstoff mit nicht-kariogener Eigenschaft zugleich als Geschmackskorrigens dient.

In einer besonderen Ausführungsform der Erfindung weisen Pellets aus Matrixmassen mit Weichmacherzusätzen von 20 bis 50% (bezogen auf die zu verarbeitende Masse) ausgeprägte bioadhäsive Eigenschaften auf.

Weiterhin kann es wünschenswert sein, den beschriebenen Matrixmassen lipophile Bestandteile, wie z.B. Phospholipidezur Ausbildung von Liposomen zuzusetzen.

Für Pellets als Formkörper, die sich in Wasser bei 37°C innerhalb weniger Minuten auflösen, werden bevorzugt Gelatinesorten ausgewählt, deren Peptidanteil oberhalb 30% liegt und die ein Maximum der Molekulargewichtsverteilung bei ca. $10^5$ D bis $10^6$ D aufweisen.

Als zusätzliche Gerüstbildner von 1-50% (bezogen auf die zu verarbeitende Masse) können eingesetzt werden: Albumine, Agar-Agar, Gummi arabicum, Pektine, Traganth, Xanthan, natürliche sowie modifizierte Stärken, Dextrane, Dextrine, Maltodextrin, Chitosan, Alginate, Alginat-Calciumphosphate, Cellulosederivate, Polyvinylalkohol, Polyvinylpyrrolidon, Polyacrylsäure und Polymere aus Methacrylsäure und Methacrylsäureestern.

Celluloseacetatphtalat oder Hydroxypropylmethylcellulosephtalat, azo-vernetzte Polymethacrylate; Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden; Galactomannanderivate wie Ethyl- oder Acetylgalactomannane; mit Adipinsäure vernetzte Polysaccharide, lipophile Substanzen wie abbaubare Mono-, Di-, und Triglyceride; erodierbare Fettalkohole.

In einer weiteren Ausführungsform der Erfindung können 1-50 % ige Zusätze von Stoffen aus dieser Gruppe ausgewählt werden, um die physikalischen oder chemischen Eigenschaften der Matrix wie z.B. die Viskosität, die mechanische Festigkeit oder die Auflöseeigenschaften des polymeren Gerüstes auf den Wirkstoff und den Anwendungszweck abzustimmen. So können beispielsweise Stoffe wie Dextrane, modifizierte Stärken, Zucker und insbesondere Mannit erfindungsgemäß Pellets hergestellt werden, die als Lyophilisat ein hochporöses Netzwerk ausbilden. Makromoleküle wie z.B. Alginate, Agar-Agar, Pektine können erfindungsgemäß zur zusätzlichen Verzögerung oder Modifizierung der Wirkstofffreigabe dienen.

Zu dieser Grundmasse können weitere, für pharmazeutische Anwendung geeignete Hilfs- und Trägerstoffe, wie z.B. Füllstoffe, wie z.B. Lactose, Dispergiermittel, wie z.B. Dinatriumphosphat, pH-Korrigentien, wie z.B. Dinatriumcitrat, Emulgatoren, wie z.B. Lecithin, Stabilisatoren, wie z.B. Ascorbinsäure, Kosolventien, wie z.B. Polyethylenglycol, natürliche Farbstoffe, wie z.B. Carotinoide, aromatisierende Stoffe oder Geschmackskorrigentien, wie z.B. Zuckerersatzstoffe, Komplexbildner oder Einschlußkomplexbildner, wie z.B Cyclodextrin zugesetzt werden.

In einer besonderen Ausführungsform der in Variante A und B angegebenen Matrixmassen bzw. Mischungen, die mit oder ohne Weichmacherzusatz aufgebaut sein können, kann durch Zusatz von magensaftresistenten Stoffen aus der Gruppe: Poly- und Methacrylsäurederivate, Cellulosederivate, und deren Mischungen Pellets hergestellt werden, die den Arzneistoff erst nach der Magenpassage freisetzen, d.h. daß der Gerüstbildner der Matrix-Mischung sich in einem vorbestimmten pH-Bereich auflöst.

Anstatt der obengenannten magensaftresistenten Stoffe können auch Substanzen verwendet werden, die erst nach Erreichen eines bestimmten Darmabschnittes durch dort vorhandene Enzyme abgebaut werden. Hierbei handelt es sich z.B. um azo-vernetzte Polymethacrylate; Polyurethan-Zucker-Copolymere, wobei als Zuckerkomponente insbesondere oligomere Galactomannane oder Galactomanndeerivate geeignet sind, die dann mit aliphatischen Diisocyanaten vernetzt werden; Galactomannanderivate wie Ethyl- oder Acetylgalactomannane; mit Adipinsäure vernetzte Polysaccharide.

Auf diese Weise sind erfindungsgemäß Pellets herstellbar, die sich insbesondere für Colonarzneiformen eignen. Nach Erreichen des Colons wird eine derartige Pelletmatrix enzymatisch abgebaut und der inkorporierte Arzneistoff damit gezielt in diesem gastrointestinalen Abschnitt freigegeben.

Weitere Ausführungen zu Colonararzneiformen finden sich insbesondere in der "Peptidarzneistoffe enthaltende Formkörper und ihre Herstellung sowie deren Verwendung" betitelten internationalen PCT-Anmeldung PCT/DE00036 (WO 93/13753) der ALFATEC-Pharma GmbH vom gleichen Tage.

Im Falle von alginathaltigen Grundrezepturen kann man durch Suspendierung von wasserunlöslichem Dicalciumhydrogenphosphat ($(Ca_2(HPO_4)_2)$ z.B. zu einer pH-neutralen bis leicht basischen Gelatine/Alginat-Mischung Pellets erzeugen, die den Wirkstoff verzögert freigeben. Während der Magenpassage löst das saure Medium das Calciumsalz auf und vernetzt das Alginat.

Da die die erfindungsgemäßen Pellets, als Formkörper, hohe mechanische Stabilität besitzen, können sie mit pharmazeutisch gebräuchlichen Filmbildnern überzogen werden. Besonders vorteilhaft kann durch Kombination von Matrixmassen, die insbesondere bioadhäsive Eigenschaften aufweisen, und Filmüberzügen (z.B. Poly-und Methacrylsäurederivate), die sich in definierten pH-Bereichen auflösen, gezielt der gewünschte Resorptionsabschnitt im gastrointestinalen Trakt erreicht werden.

Solche bioadhäsiven Eigenschaften können beispielsweise durch Teilvernetzung einer Matrix erzeugt werden, die aus einem von Kollagen abgeleiteten Hilfsstoff aufgebaut ist.

Statt Eudragiten[R] können auch geeignete Filmüberzüge aus Substanzen, die nach Erreichen des Colons durch dort vorhandene Enzyme abgebaut werden, eingesetzt werden. Hierbei handelt es sich z.B. um azo-vernetzte Polymethacrylate; Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden; Galactomannanderivate wie Ethyl- oder Acetylgalactomannane; mit Adipinsäure vernetzte Polysaccharide.

Diese Vorgehensweise ermöglicht es, Arzneistoffe kontrolliert zur Resorption zu bringen. Weiterhin können durch Kombinationen von Filmbildnern Pelletmischungen erfindungsgemäß hergestellt werden, die den Wirkstoff aus der Arzneiform gepulst freisetzen.

Bekanntermaßen besitzt Gelatine je nach Herstellungsverfahren einen isoelektrischen Punkt im sauren (Gelatine Typ B) oder im alkalischen Bereich (Gelatine Typ A). Diese Eigenschaft wird erfindungsgemäß zur direkten Bildung von Mikro- bzw. Nanokapseln in der Matrixmasse ausgenutzt. So können bei Verwendung von Gelatinen mit entgegengesetzter Ladung in Mischung mit wirkstoffhaltiger Lösung (z.B. bei einem pH von 6-7) durch Entfernung des Lösungsmittels Mikrokapseln hergestellt werden. Bei Verwendung von Gelatinesorten oder Kollagenderivaten mit definierter molekularer Zusammensetzung lassen sich dreidimensionale Vernetzungen im Nanometerbereich durchführen. Gelatinen oder Kollagenhydrolysate können weiterhin mit dem Wirkstoff z.B. unter einem ca. 2-3%igen Zusatz von Salzen Konjugate bilden.

Die eingangs beschriebene, erfindungsgemäße Bioverfügbarkeitssteigerung von Arzneistoffen läßt sich erstaunlicherweise bereits erzielen, wenn ein Arzneistoff in <u>grobdisperser Form</u> in einer Pelletmatrix dispergiert vorliegt.

Bei Verwendung von mikronisierten Pulvern, die in einer erfindungsgemäßen Pelletmatrix dispergiert vorliegen, ergibt sich im Vergleich zu einer herkömmlichen Suspension eines mikronisierten Pulvers nochmals eine deutliche Bioverfügbarkeitssteigerung. Offensichtlich führt das Vorliegen eines Arzneistoffs in einer erfindungsgemäßen Zubereitung vorteilhafterweise zu einer sehr stark erhöhten (effektiveren) Resorption des Arzneistoffs unter physiologischen Bedingungen.

Durch Kombination von Pellets, die Wirkstoffe aus unterschiedlichen Indikationsgruppen enthalten, lassen sich Kombinationspräparate erhalten, z.B. durch Abfüllen in übliche Hartgelatinekapseln.

Andere Anwendungszwecke sind z.B. das Abfüllen in Beutel zu Trinkgranulaten (-pellets) oder die Verwendung zur Bereitstellung von Initialdosen in Retardarzneiformen etc.

Von einem einzigen Produkt - den erfindungsgemäßen Formkörpern - ausgehend ist damit eine beträchtliche technologische Anwendungsbreite gegeben.

Im folgenden wird das verfahren zur Herstellung der erfindungsgemäßen Pellets näher beschrieben.

Weitere Ausführungen hierzu sind in den im folgenden aufgelisteten parallelen internationalen (PCT)-Anmeldungen enthalten. Die Inhalte dieser parallelen PCT-Anmeldungen, am selben Tage beim Deutschen Patentamt von denselben Erfindern und Anmeldern eingereicht:

    PCT/DE93/00037 = WO93/13754
    PCT/DE93/00035 = WO93/13761
    PCT/DE93/00036 = WO93/13753

werden hiermit ebenso vollinhaltlich zur Offenbarung der vorliegenden Anmeldung gemacht, wie die älteren PCT-Anmeldungen:
PCT/DE92/01010,                PCT/DE92/01012,
PCT/DE92/01014,                PCT/DE92/01016,
PCT/DE92/01007,                PCT/DE92/01008,
PCT/DE92/01015,                PCT/DE92/01013,
PCT/DE92/01009, PCT/DE92/01011 vom 4.12.1992.

Im einfachsten Fall läßt sich das erfindungsgemäße Verfahren zur Herstellung von Wirkstoff-enthaltenden Festkörpern mit den folgenden Verfahrensschritten beschreiben:

    a) Man löst einen Gerüstbildner aus hydrophilen Makromolekülen in einem Lösungsmittel,

    b) man dispergiert in dieser Lösung den Wirkstoff,

    c) man tropft die erhaltene Mischung aus gelöstem Gerüstbildner und dispergiertem Wirkstoff in ein tiefkaltes inertes verflüssigtes Gas ein und bildet damit den Festkörper aus, und

    d) Man trocknet die so geformten Pulver, Granulate oder Pellets durch Verdampfen oder Sublimieren des Lösungsmittels auf übliche Weise.

Der erste Schritt des Verfahrens besteht darin, daß man das hydrophile Makromolekül, insbesondere Gelatine, fraktionierte Gelatine, Kollagenhydrolysate oder Gelatinederivate oder auch Mischungen von makromolekularen Stoffen in einem geeigneten Lösungsmittel - Wasser als Lösungsmittel der Wahl ist in den meisten Fällen zu bevorzugen - löst. Dabei kann Anwendung von Wärme erforderlich sein, wie z.B. bei Gelatine eine Temperatur von 37°C oder mehr, um ein Gelatinesol zu erhalten.

Weitere Hilfs- und Trägerstoffe, wie z.B. Füllstoffe, wie z.B. Lactose, Dispergiermittel, wie z.B. Dinatriumhydrogenphosphat, pH-Korrigentien, wie z.B. Dinatriumcitrat, Emulgatoren, wie z.B. Lecithin, Stabilisatoren, wie z.B. Ascorbinsäure, Kosolventien, wie z.B. Polyethylenglycol, natürliche Farbstoffe, wie z.B. Carotinoide, aromatisierende Stoffe oder Geschmackskorrigentien, wie z.B. Zuckerersatzstoffe, Komplexbildner oder Einschlußkomplexbildner, wie z.B Cyclodextrin zugesetzt werden.

Konzentrationsbereiche der hydrophilen Makromoleküle, insbesondere Gelatine, Kollagenhydrolysate oder Gelatinederivate liegen bevorzugt unterhalb von 30% (Gewichtsprozent), z.B. im Bereich von 3 - 15%, bezogen auf die zu verarbeitende Masse ohne Wirkstoff. Entsprechend beträgt der Wassergehalt der zu verarbeitenden Masse bis zu ca. 70 Gew.-% oder mehr.

Konzentrationsbereiche der zusätzlichen Gerüstbildner, wie beispielsweise Dextrane, Saccharose, Glycin, Lactose, Polyvinylpyrrolidon, insbesondere aber Mannit liegen unterhalb von 30% (Gewichtsprozent), z.B. im Bereich von 0 - 15%, bezogen auf die zu verarbeitende Masse ohne Wirkstoff. Vorzugsweise ist der Anteil an zusätzlichem Gerüstbildner nicht größer als der Anteil an dem eigentlichen Gerüstbildner.

Diese Stoffe, insbesondere aber Mannit, können als Füllkomponenten die Stabilität des polymeren Gerüsts in den erfindungsgemäßen Pellets verbessern und somit auch deren mechanische Eigenschaften.

Im zweiten Schritt wird der Wirkstoff in der Lösung eines hydrophilen Makromoleküls gemäß Stufe 1 gelöst oder suspendiert.

Das im zweiten Schritt beschriebene System wird nun im dritten Schritt zur Formgebung über ein geeignetes Dosiersystem in eine tiefkalte, leicht verdampfbare Flüssigkeit eingetropft, bevorzugt in ein Tauchbad mit flüssigem Stickstoff. Jeder diskrete Tropfen nimmt dabei einerseits bereits während des freien Falls, andererseits im Tauchbad durch die um ihn entstehende Gashülle bzw. die Grenzflächenspannung System/Gas Kugelgestalt an, bevor ein vollständiges Ausfrieren erfolgt. Gerade dieses schnelle, aber dennoch kontrolliert steuerbare Gefrieren fixiert den gegebenen Zustand des Systems augenblicklich, d.h. es kann kein Arzneistoff in das umgebende Medium diffundieren, gelöster Arzneistoff kann nicht mehr auskristallisieren, Suspensionen können nicht mehr sedimentieren, thermisch empfindliche oder feuchtigkeitsempfindliche Stoffe werden cryokonserviert, das Trägergerüst kann nicht zusammenschrumpfen usw. Das Herstellungsverfahren mit einem inerten Flüssiggas hat also keine nachteilige Beeinflussung oder Veränderung des Produkts zur Folge, was einen großen Vorteil darstellt. Die gewünschten Eigenschaften werden beibehalten.

Bei einer Ausführungsform des unter a) beschriebenen Verfahrensschrittes stellt man eine tropffähige Masse, vorwiegend aus hydrophilen Makromolekülen als Gerüstbildner, insbesondere Pflanzenproteine, Pflanzenproteinhydrolysate, Kollagen, Gelatine, fraktionierter Gelatine, Elastinhydrolysate, Kollagenhydrolysaten, Gelatinederivaten oder Mischungen der vorgenannten Stoffe und dem Wirkstoff her.

Zunächst dispergiert, d.h. löst, oder suspendiert man den Wirkstoff, z.B. in dem gelöst vorliegenden Gerüstbildner, insbesondere Pflanzenproteine, Pflanzenproteinhydrolysate, Kollagen, Gelatine, fraktionierte Gelatine, Gelatinederivate, Kollagenhydrolysate oder Elastinhydrolysat, wobei die Art und Menge des eingesetzten Gerüstbildners und gegebenenfalls der Zusatz von weiteren Hilfsstoffen vom späteren Verwendungszweck der Formkörper abhängt. Die Konzentration des Trägermaterials kann beispielsweise von 0,5 bis 60% (g/g), bevorzugt 0,5 bis 30% (bezogen auf die zu verarbeitende Masse) variieren. Die Anwendung von Wärme im Temperaturbereich von ca. 30°C bis 60°C, vorzugsweise ca. 45°C, kann z.B. beim Einsatz von Gelatine erforderlich sein, um diese in die Solform zu überführen.

Ein Zusatz von zusätzlichen Gerüstbildnern von 1-50% (bezogen auf die zu verarbeitende Masse) ausgewählt aus der Gruppe bestehend aus: Albuminen, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylalkohol, Polyvinylpyrrolidon, Polyacrylsäure und Polymere aus Methacrylsäure und Methacrylsäureestern, Celluloseacetatphtalat oder Hydroxypropylmethylcellulosephthalat, azo-vernetzte Polymethacrylate; Polyurethan-Zucker-Copolymere, wobei als Zuckerkomponente insbesondere oligomere Galactomannane oder Galactomannanderivate geeignet sind, die dann im aliphatischen Diisocyanaten vernetzt werden; Galactomannanderivate wie Ethyl- oder Acetylgalactomannane; mit Adipinsäure vernetzte Polysaccharide; lipophile Substanzen wie abbaubare Mono-, Di-, und Triglyceride; erodierbare Fettalkohole kann weiterhin der Matrixmasse zugegeben werden.

In einer weiteren Verfahrensvariante können der Matrix Zusätze von Weichmachern von 1-50% (bezogen auf die zu verarbeitende Masse) ausgewählt aus der Gruppe bestehend aus: Glycerol, Propylenglykol, Polyethylenglykole, Triacetin, Sorbitol, Sorbitangemische, Sorbitollösungen, Glucosesirup und andere Polyole bzw. Zuckeralkohole beigefügt sein.

Zu dieser Grundmasse können weitere für pharmazeutische Anwendung geeignete Hilfs- und Trägerstoffe, wie z.B. Füllstoffe, wie z.B. Lactose, Dispergiermittel` wie z.B. Dinatriumhydrogenphosphat, pH-Korrigentien, wie z.B. Dinatriumcitrat, Emulgatoren, wie z.B. Lecithin, Stabilisatoren, wie z.B. Ascorbinsäure, Kosolventien, wie z.B. Polyethylenglycol, natürliche Farbstoffe, wie z.B. Carotinoide, aromatisierende Stoffe oder Geschmackskorrigentien, wie z.B. Zuckerersatzstoffe, Komplexbildner oder Einschlußkomplexbildner, wie z.B Cyclodextrin zugesetzt werden.

Selbstverständlich eignen sich die erfindungsgemäßen Mischungen zu einer sofortigen Abfüllung in flüssiger Form nach dem unter a) beschriebenen Verfahrensschritt zur Ausformung in Behältnissen, wie z. B. Formen, Weichgelatinekapseln sowie geeignete andere Umhüllungen.

In einer Ausführungsform des unter c) beschriebenen Verfahrensschrittes wird die beschriebene Matrixmasse zur Ausrundung (Formgebung) und Schockfrostung in ein Tauchbad im Bereich von ca. -70°C bis ca. -270°C, vorzugsweise von ca. -100°C bis -220°C eingetropft. Als tiefkalte, insbesondere inerte, Flüssigkeit wird vorzugsweise flüssiger Stickstoff eingesetzt, der die Bestandteile der Pellets nicht verändert. In der tiefkalten Flüssigkeit bilden sich runde Formkörper (Pellets), die nach der Trocknung eine mechanisch stabile Matrix ausbilden. Die Formgebung erfolgt über ein geeignetes Dosiersystem. Von besonderem Vorteil ist somit der Erhalt der gewünschten Eigenschaften. Weiterhin arbeitet das Verfahren lösungsmittelfrei, belastet die Umwelt nicht und kann unter Sterilbedingungen durchgeführt werden.

Als Dosiersysteme eignen sich alle Vorrichtungen, die diskrete, gleichmäßige Gebilde, z. B. Tropfen, vorherbe-stimmbarer Größe erzeugen können.

Verwendet man z.B. ungeregelte Tropfvorrichtungen, so erhält man Granulate, bei Verwendung von geeigneten Sprüh- oder Zerstäubungsdüsen mit Dosierpumpen erhält man bevorzugt Pulver als Formkörper.

Weiterhin können für das erfindungsgemäße verfahren Dosiervorrichtungen mit Düsen, die das zu tropfende Gut getaktet oder intermittierend ausstoßen, verwendet werden.

Eine weiterhin bevorzugte Ausführungsform des erfindungsgemäßen Verfahrens setzt das von Messer Griesheim GmbH entwickelte Cryopel 《Verfahren (basierend auf DE-OS 37 11 169) ein. In Verbindung mit einer Tauchfrostanlage, der CryopelR-Anlage, ist die apparative Umsetzung des erfindungsgemäßen Verfahrens in den industriellen Maßstab besonders einfach. Diese Anlage, die mit flüssigem Stickstoff betrieben werden kann, zeichnet sich besonders durch ihre Wirtschaftlichkeit aus. Diese Anlage ist auch für Sterilherstellung geeignet. Kontinuierliche Arbeitsweise bei geringem Wartungs- und Reinigungsaufwand ermöglicht die wirtschaftliche Umsetzung des erfindungsgemäßen Verfahrens in den industriellen Maßstab.

Es zeigt:

Fig. 1: eine schematische Darstellung in Schnittansicht einer Vorrichtung zur Ausführung des erfindungsgemäßen Verfahrens; und
Fig. 2: eine weitere Ausführungsform einer Vorrichtung zur Durchführung des erfindungsgemäßen Verfahrens in schematischer Darstellung.
Fig. 3: schematisch die Vorgänge, die bei der passiven Resorption von Arzneistoffen in der Gastrointestinalmembran ablaufen.

In Fig. 1 ist das von Messer Griesheim GmbH entwickelte Cryopel$^R$-Verfahren schematisch dargestellt. Die erfindungsgemäße Matrixlösung, die den Wirkstoff gelöst, emulgiert oder suspendiert enthält, wird aus der beheizbaren Eintragsvorrichtung 1 über kalibrierte Düsen in das Flüssigstickstoffbad 3 bei -196°C eingetropft und unter gleichzeitiger Schockfrostung zu runden Pellets geformt. Über das kontinuierlich über Umlenkrollen laufende Transportband 2 wird das gefrorene Produkt über die Vorrichtung 5 ausgetragen. Die Dosierung des Flüssigstickstoffes erfolgt über die Zuleitung 7 und das entstehende Stickstoffgas entweicht über die Leitung 6. Die Isolierung 4 umschließt das gesamte System.

In Fig. 2 ist eine schematische Darstellung eines Verfahrens gezeigt, bei der über eine regelbare Dosierpumpe 8 die kalte bzw. auf max. 60°C erwärmte Wirkstoff-Matrixdispersion über die Zuleitung 9 kontinuierlich über die beheizbaren Tropfdüsen 10 in die Isolierwanne 11 mit Flüssigstickstoff 12 eintropft. Die schockgefrosteten Pellets werden chargenweise oder kontinuierlich entnommen. Mit dieser Vorrichtung lassen sich hochviskose Massen verarbeiten.

Sollte das zu verarbeitende System nicht ausreichend fließ- bzw. tropffähig sein, kann z.B. weiterer Wasserzusatz von 1-10 Gew.% erfolgen, die Verarbeitungstemperatur erhöht werden oder aber auch Druck bei der Dosierung zur Anwendung kommen. Im umgekehrten Falle (System zu niedrigviskos) ist analog Unterdruck bzw. Temperaturerniedrigung anzuwenden. Auf diese Weise gewährleistet man gleichmäßige Bildung, wie auch Abriß der einzelnen Tropfen.

Die Verarbeitungstemperatur kann in weiten Bereichen variiert werden, soll aber im Falle von thermolabilen Wirkstoffen unterhalb von 50°C liegen.

Somit können beispielsweise mit den beschriebenen Dosiervorrichtungen Massen, deren Viskosität sich in einem weiten Bereich bewegt, z.B. $1 \times 10^{-3}$ bis 12,5 Pa x s (Pascalsekunden) und höher, problemlos dosiert werden.

Weitere tiefkalte inerte verflüssigte Gase, die sich für das erfindungsgemäße Verfahren eignen, können z.B. flüssige Edelgase wie Argon sein.

In Abhängigkeit vom gewählten Dosiersystem kann eine Korngrößeneinheitlichkeit von über 80% erreicht werden, die sich durch Klassieren noch zusätzlich erhöhen läßt.

Durch Klassieren der gefrorenen und abgetrennte Anteile können diese erneut in den flüssigen Zustand überführt und wieder pelletiert werden, so daß ein verlustfreies Arbeiten gewährleistet ist.

Beim Trocknen der so erhaltenen Pellets ergeben sich zwei Verfahrensvarianten:

Verfahrensvariante A:

Die bei -196°C (flüssiger Stickstoff) gefrorenen Formkörper, z.B. Pellets, werden in eine Gefriertrocknungsanlage überführt. Dabei werden Temperaturen von 15°C unterhalb des Sublimationspunktes von Wasser bei einem Druck von 0,1 Pa bis $10^3$ Pa (0,001 bis 1,03 mbar) gewählt. Der Trocknungsvorgang, der in einer herkömmlichen Gefriertrocknungsanlage (Kondensatortemperatur -40°C) bei -25°C und 33 Pa (0,33 mbar) in der Primärtrocknung unter Sublimation des durch die Schockfrostung amorph gefrorenen Wassers aus der Matrix abläuft, führt nach Sekundärtrocknung (Desorption) zu einem Endprodukt mit einem hochporösen Netzwerk. Durch die erfindungsgemäße Schockfrostung wird das Wasser überwiegend an der Ausbildung einer kristallinen Phase gehindert, wodurch sich eine feste feindisperse amorphe Wasserphase in der Matrix ausbildet. Nach der Sublimation des derartig vorliegenden Wassers entstehen hochporöse Mikroporen-haltige Netzwerke, die gegenüber herkömmlichen Gefrierverfahren eine deutlich erhöhte Oberfläche aufweisen. Solche Pellets sind gegenüber herkömmlich gefriergetrockneter Ware besonders leicht löslich und sind bevorzugt zur Entwicklung von Instantzubereitungen geeignet.

Verfahrensvariante B:

Die gefrorenen Formkörper, z.B. Pellets, werden aufgetaut und konventionell getrocknet. Hierbei kann vorteilhaft zur Beschleunigung des Trocknungsvorgangs und zur Einhaltung von niedrigen Temperaturen unter Vakuum (ca. 3.000-5.000 Pa (ca. 30-50 mbar)) gearbeitet werden. Es können Trocknungstemperaturen von bis zu 50°C gewählt werden, wobei die Temperatur während des Trocknungsvorganges in der Pelletmatrix aufgrund der Verdampfungsenthalpie der Flüssigkeit nicht über 30°C ansteigt.

Für konventionell getrocknete Pellets (Verfahrensvariante B) sind sol-gel-bildende Substanzen für die Matrix notwendig, die in Solform tropffähig sind und nach der Cryopelletierung bzw. nach dem Auftauen ein Gel ausbilden, das nach der Trocknung stabil ist. Ein Zusatz von Weichmachern beeinflußt die Matrixmasse hinsichtlich der Konsistenz. So hergestellte Pellets zeichnen sich durch eine besonders kostengünstige Herstellung aus, da der Verfahrensschritt der Lyophilisation nicht unbedingt notwendig ist.

Bei Pflanzenextrakten, deren Wirkkomponenten sowohl hydrophile als auch lipophile Eigenschaften zeigen, werden die lipophilen Anteile zunächst in der Matrixmasse emulgiert und die wasserlöslichen Bestandteile in der hydrophilen Matrixmasse aufgelöst und anschließend cryopelletiert.

Bedingt durch die erhöhte Viskosität des Matrixsystems können suspendiert vorliegende Wirkstoffe durch einfaches Rühren an der Sedimentation gehindert und gleichmäßig dosiert werden. Temperaturempfindliche Arzneistoffe werden vorteilhaft lyophilisiert.

Die Verarbeitung der in den Unteransprüchen angegebenen besonderen Ausbildungsformen der Erfindung wie z.B. Formulierungen mit kontrollierter Freigabe bzw. Resorptionsverbesserung, Mikro- und Nanoverkapselung, Ausfällungen, Konjugatbildung, Filmüberzüge und die Herstellung von Pellets mit bioadhäsiven Eigenschaften erfolgt sinngemäß der Beschreibung und in Abstimmung auf den jeweiligen Wirkstoff.

Das erfindungsgemäße Verfahren selbst ist gegenüber dem Stand der Technik insgesamt gesehen wartungsarm und wirtschaftlich durchzuführen. Die an sich einfach durchzuführende Cryopelletierung ermöglicht auf überraschende Weise den Stand der Technik deutlich zu überragen.

Zur Durchführung des erfindungsgemäßen Verfahrens genügt es im einfachsten Fall, eine wäßrige Gelatinelösung mit einer Gelatinesorte der bezeichneten Spezifikation herzustellen, darin das Nifedipin bzw. das Dihydropyridinderivat in fein kristalliner Form homogen zu suspendieren, und das System über eine geeignete Dosiervorrichtung in ein Tauchbad mit flüssigem Stickstoff zu tropfen. Die auf diese Weise geformten, tiefgefrorenen Pellets werden anschließend durch Lyophilisation in den trockenen Zustand überführt.

Im Rahmen der vorliegenden Erfindung hat sich vorteilhaft gezeigt, daß feindisperse Werkstoff-Fällungen auch durch Präzipitation aus einer Lösung des Wirkstoffs in einem mit Wasser mischbaren und pharmazeutisch akzeptablen organischen Lösungsmittel, wie z. B. Alkohol, direkt in der Gelatinelösung erzeugt werden können. Nach Entfernen des Alkohols (z. B. durch Evaporation) verfährt man analog zu der beschriebenen Verfahrensweise, um die erfindungsgemäßen Formkörper herzustellen.

Im Falle von optisch aktiven Substanzen lassen sich sowohl deren Racemate, wie auch die enantiomerreinen Komponenten und Mischungen davon einsetzen.

Bedingt durch die große Variationsbreite der Erfindung können in den beschriebenen Matrixmassen alle Arzneistoffe enthalten sein, sofern sie keine Inkompatibilitäten mit den einzelnen Bestandteilen der Rezepturmassen zeigen. Der Begriff Arzneistoff wird dabei erfindungsgemäß wie folgt definiert:

Arzneistoffe können synthetischen oder natürlichen Ursprungs sein, sowohl chemisch einheitliche Substanzen oder Substanzgemische sein, als auch Kombinationen aus verschiedenen pharmakologisch wirksamen Komponenten. Ferner soll der Arzneistoffbegriff aber auch Phytopharmaka und Pflanzenextrakte allgemein umfassen und schließlich auch Hormone, Vitamine und Enzyme mit einbeziehen.

Ein Pflanzenextrakt wird in der Regel aus der folgenden Gruppe ausgewählt: feste Pflanzenextrakte, flüssige Pflanzenextrakte, hydrophile Pflanzenextrakte, lipophile Pflanzenextrakte, einzelne Pflanzeninhaltsstoffe; sowie deren Mischungen.

Auch enantiomerreine Wirkstoffe oder Pseudoracemate sind erfindungsgemäß geeignet.

Weiterhin können Wirkstoffe aus dem Bereich der diätetischen Lebensmittel (health care) sowie aus dem Bereich der Kosmetik verwendet werden.

Im Falle für die Erfindung geeigneter Arzneistoffe besteht keinerlei Begrenzung bzgl. der Indikationsgruppen. Im folgenden werden beispielhaft Indikationsgruppen und einige zugehörige Vertreter genannt:

starke Analgetika, Antirheumatika/Antiphlogistika (NSAR), beta-Sympathicolytica, Steroidhormone, Tranquillizer, alpha-Sympathicolytica, Hypnotika und Sedativa, tricyclische Antidepressiva, Neuroleptika, Gichtmittel, Antiparkinsonmittel, Koronartherapeutika oder Calciumantagonisten, Antihypertensiva, Diuretika, orale Antidiabetika; Chemotherapeutika oder Antibiotika, Lokalanästhetika, ACE-Hemmstoffe, Mukolytika, Antiasthmatika, Mineralstoffpräparate, Neurotropika, Ulcustherapeutika, Provitamine und Vitamine, Peptidarzneistoffe, Digitalisglykoside, Antiemetika, Enzyme, Antiarrhytmika, Antiepileptika, Antikoagulantia, Spasmolytika, Antimykotika, Hormone, Venentherapeutika, Immunsuppresiva, Tuberkulostatika, Virustatika, Zytostatika, Impfstoffe, Phytopharmaka, Stoffe zur Behandlung von AIDS, Calciumantagonisten.

Gegenüber dem Stand der Technik lassen sich besonders vorteilhaft Wirkstoffe mit schlechter Verträglichkeit, sowie licht-, oxidations-, hydrolyse- und temperaturempfindliche Stoffe wie z.B. Peptide, Naturstoffe, Enzyme, Vitamine etc. zu Arzneiformen erfindungsgemäß verarbeiten.

Um die physiologischen Hintergründe der Resorption von Arzneistoffen im allgemeinen und die verbesserte Resorptionsquote der erfindungsgemäßen Pelletformulierungen ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:

a) die Gastrointestinalmembran wirkt als Lipidbarriere,
b) der Arzneistoff wird nur in gelöster und ungeladener,
d.h. nichtionisierter Form aufgenommen,
c) saure Arzneistoffe werden bevorzugt im Magen, basisische Arzneistoffe bevorzugt im Darm resorbiert.

Hydrophile Makromoleküle, insbesondere Gelatine, sind amphiphile Substanzen, die je nach pH-Wert unterschiedliche Ladungszustände aufweisen. Erfindungsgemäß kann nun das hydrophile Makromolekül in den erfindungsgemäßen Systemen so ausgewählt werden, bzw. der pH-Wert der Formulierung so abgestimmt werden, daß sich im physiologischen Milieu ein positiver Ladungszustand ergibt. Damit läßt sich zumindest eine Teilneutralisation der negativen Oberflächenladungen der Glycocalix erreichen. Dieses Neutralisationsphänomen kann durch bioadhäsive Eigenschaften des hydrophilen Makromoleküls, insbesondere Gelatine noch verstärkt wirksam werden.

Da gelöste Arzneistoffmoleküle nun die Glykocalix ungehindert passieren können, ohne durch elektrostatische Effekte gebunden bzw. abgestoßen zu werden, erreichen sie damit auch die Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption liefern.

Die Verwendung der erfindungsgemäßen Pulver, Granulate oder Pellets als Formkörper kann z.B. über übliche Dosiersysteme in Hartgelatinekapseln oder als Granluat in Beutel erfolgen. Durch die gute Rieselfähigkeit bzw. annähernd runde Form der Granulate läßt sich eine gute Dosierbarkeit gewährleisten. Bei Verwendung von Pellets ist die dichteste Kugelpackung in genauer Abstimmung des Schüttvolumens auf die Kapselgröße möglich, woraus eine Verbesserung der Dosiergenauigkeit beim Abfüllvorgang resultiert. Darüberhinaus kann durch die entsprechende Auswahl einer bestimmten Pelletgröße auf den Zusatz von Füllstoffen verzichtet werden.

Die Pellets mit einer Größe von 2-12 mm können erfindungsgemäß für eine neue einzeldosierte bukkale, nasale oder perorale Arzneiform verwendet werden. Peroral eingesetzte Pellets sind leicht schluckbar und können in Gläsern mit Dosierspendern umweltschonend abgegeben werden. Bei bukkaler und nasaler Verwendung eignen sich Formkörper-Pellets mit bioadhäsiven Eigenschaften.

Pulver, Granulate oder Pellets -als Formkörper- aus Matrixmassen, die sich in kaltem Wasser schnell und vollständig auflösen, können -in Beuteln abgefüllt- als Instantzubereitungen für den pharmazeutischen oder diätetischen Bereich (health care) verwendet werden.

Überraschenderweise können unter Ausnutzung der bioadhäsiven Eigenschaften der Sol-Gel-Bildner, insbesondere Gelatine, mit den erfindungsgemäßen Formkörpern bukkale und nasale Formulierungen bzw. Arzneiformen mit pH-gesteuerter Freigabe zur Anwendung kommen.

Eine weitere Verwendung dieser speziellen Granulate oder Pellets als Formkörper ist durch ihre direkte Verpreßbarkeit zu Tabletten gegeben. Die so erhaltenen Tabletten zeigen, bei geringer Friabilität und hoher Bruchfestigkeit, erstaunlicherweise eine völlige Auflösung innerhalb von 5 Minuten, z.B. 2 Minuten, gemessen nach üblichen Testmethoden (z.B. Dissolutiontestapparatur gemäß USP). Überraschenderweise bleiben die guten Auflösungseigenschaften der Gerüstmatrix auch nach dem Komprimieren erhalten. Die Tabletten lösen sich ohne vorgelagerten Zerfall direkt auf. Im Gegensatz dazu zerfallen aus herkömmlichen Granulaten verpreßte Tabletten zuerst immer in Granulatteilchen, die sich erst anschließend auflösen.

Die Tablettenherstellung aus erfindungsgemäßen, gefriergetrockneten Formkörpern ist beispielsweise bei der Konzipierung einer Arzneiform für temperaturempfindliche Wirkstoffe von Bedeutung. Solche Arzneistoffe erfordern wegen ihrer Empfindlichkeit (z.B. Hitzeinaktivierung usw.) besonders schonende Verarbeitungsprozesse, die vorteilhafterweise durch das erfindungsgemäße Verfahren sehr leicht und einfach sichergestellt werden können.

Der Anwendungsbereich für die erfindungsgemäßen Formkörper ist selbstverständlich nicht nur auf pharmazeutische Zwecke beschränkt. Einsatzgebiete können auch auf kosmetischem Gebiet liegen (Verarbeitung von Planzenextrakten wie z.B. Aloe vera zu Pellets bietet den Vorteil einer idealen, trockenen Transportform für den feuchteempfindlichen Extrakt und zugleich ist das natürlich aufgebaute Matrixsystem als Bestandteil für Salben und Cremes besonders geeignet).

Bedingt durch die vielfältigen Variations- und Kombinationsmöglichkeiten der erfindungsgemäßen Formkörper läßt sich die Freisetzung von Arzneistoffen in allen angegebenen Verwendungszwecken in weiten Grenzen modulieren.

Die folgenden Beispiel sollen die Erfindung näher erläutern:

Beispiel 1:

Arzneistoff: Kaliumchlorid
Rezeptur der zu verarbeitenden Grundmasse:
625 g Kollagenhydrolysat (Molekulargewicht 2.000-3.000 D)
50 g Citronensäure
2325 g destilliertes Wasser
3000 g

Das Kollagenhydrolysat und die Citronensäure werden unter Rühren im Wasser gelöst. In dieser Lösung werden 190 g Kaliumchlorid gelöst.
Nach Entschäumen im Vakuum tropft man über die Cryopel -Dosiervorrichtung in ein Tauchbad mit flüssigem Stickstoff und formt damit Pellets von durchschnittlich 4 mm Größe.
Durch anschließende Gefriertrocknung wird das Wasser wie in Beispiel 1 entzogen.
Die Pellets werden in luftdichte Beutel verpackt, entsprechend einer Einzeldosierung von 1 g Kaliumionen.
Der Inhalt eines Beutels löst sich in Wasser von Raumtemperatur innerhalb von 30 sec vollständig auf.

Beispiel 2:

Arzneistoff: Phenoxymethylpenicillin-Kalium
Rezeptur der zu verarbeitenden Grundmasse:
200 g Dextran (Molekulargewicht ca. 60000)
200 g Kollagenhydrolysat (Molekulargewicht 2.000-3.000)
5 g Orangenaroma
250 g Mannit
100 g Saccharose
destilliertes Wasser ad 2500g

Die Bestandteile werden gemischt und in dem Wasser gelöst. 100 g Phenoxymethylpenicillin-Kalium werden in dieser Lösung unter Rühren gelöst.
Nach Entschäumen im Vakuum tropft man über die Cryopel -Dosiervorrichtung in ein Tauchbad mit flüssigem Stickstoff und formt damit Pellets. Durch anschließende Gefriertrocknung wird das Wasser entzogen.
2,31 g der getrockneten Pellets (entsprechend einem durchschnittlichen Gehalt an Phenoxymethylpenicillin-Kalium von 270 mg) und werden - in Einzelbeutel eingesiegelt - als Instanttrinklösung verwendet.

Beispiel 3:

Beispiel für eine Matrixmasse aus Gelatine und Weichmacher, in der zu verarbeitender Arzneistoff gelöst werden kann.

Gelatine 150 Bloom 2,6 kg
Sprühgetrocknetes Sorbitol 1,0 kg
Dihydrocodeinhydrogentartrat 0,1 kg
Wasser 6,3 kg

Der Wirkstoff wird in 1 kg Wasser unter Rühren vollständig gelöst. Das Gelatinegranulat wird in der verbleibenden Menge Wasser vorgequollen, bei 40°C aufgelöst und danach unter Rühren Sorbitol und die Wirkstofflösung zugegeben. Nach Schmelzen der Gelatine und homogenisieren der Lösung werden wie in Beispiel 1 beschrieben Pellets durch Eintropfen der Masse in flüssigen Stickstoff hergestellt. Die Pellets werden auf übliche Weise bei Temperaturen zwischen 20°C bis 40°C getrocknet und anschließend in opake Hartgelatinesteckkapseln mit einem durchschnittlichen Gehalt von 10 mg Dihydrocodeintartrat abgefüllt. Im Dissolutiontest (Apparatur nach USP XXI, 500 ml Wasser, 37°C, 50 UpM) setzt die Arzneiform 70% des Wirkstoffs in 4,5 Minuten frei.
Die erhaltenen Pellets sind durchsichtig klar und glänzend.

Beispiel 4:

Beispiel für eine Matrixmasse aus Gelatine und Weichmacher, in der der Arzneistoff emulgiert vorliegt.

Gelatine 210 Bloom 2,6 kg
Glycerin (85%ig) 1,25 kg
a-Tocopherolacetat 0,25 kg
Wasser 6,9 kg

Die pulverförmige Gelatine wird 40 Minuten in kaltem Wasser vorgequollen und anschließend bei 50°C gelöst. Mit einem Ultraschallhomogenisator wird der Wirkstoff bei 50°C in der Gelatinelösung emulgiert. Die Öl in Wasser Emulsion wird anschließend mit Glycerin vermischt und cryopelletiert. Die erhaltenen Pellets werden wie in Beispiel 4 getrocknet. Die Pellets werden mit einem Gehalt von 25 mg alpha-Tocopherolacetat in opake Hartgelatinesteckkapseln dosiert.
Die erhaltenen Pellets sehen opak undurchsichtig und glänzend aus.

Beispiel 5:

Beispiel für eine einzeldosierte Arzneiform.
Ansatz:

0,8 kg Gelatine 250 Bloom
0,8 kg sprühgetrocknetes Sorbitol
0,8 kg Acetylsalicylsäure
1,6 kg Wasser

Das Gelatinegranulat wird in dem Wasser 30 Minuten vorgequollen und anschließend bei 70°C aufgelöst. Die Acetylsalicylsäure wird in der erhaltenen Lösung dispergiert und anschließend das Sorbitol zugegeben.

Die erhaltene Matrixmasse wird über die in Fig. 2 dargestellte Apparatur bei einer Temperatur der Düsen von 70°C in flüssigen Stickstoff eingetropft. Die schockgefrosteten Pellets werden unter Kühlung klassiert und weisen eine einheitliche Größe von 8 mm auf.

Die runden Formkörper werden in einen Dosierspender abgefüllt und können - je nach Indikation - individuell dosiert werden.

So hergestellte Pellets sind wohlschmeckend und steigern die Verträglichkeit, insbesondere bei der Herzinfarktprophylaxe.

## Patentansprüche

1. Verfahren zur Herstellung von wenigstens einen wasserlöslichen Wirkstoff enthaltenden Pulvern, Granulaten oder Pellets, dadurch gekennzeichnet, daß man

   a) einen Gerüstbildner aus hydrophilen Makromolekülen ausgewählt aus der Gruppe bestehend aus:
   Kollagen, Gelatine, fraktionierte Gelatine, Kollagenhydrolysate, Gelatinederivate, Pflanzenproteine, Pflanzen-proteinhydrolysate, Elastinhydrolysate, in einem wäßrigen oder wäßrig-organischen Lösungsmittel löst,

   b) den Wirkstoff in der Lösung gemäß a) löst oder suspendiert und

   c) die erhaltene Mischung aus gelöstem Gerüstbildner und gelöstem oder suspendiertem Wirkstoff in ein tiefkaltes inertes verflüssigtes Gas eintropft und so Pulver, Granulate oder Pellets formt, und

   d) die so geformten Pulver, Granulate oder Pellets durch Verdampfen oder Sublimieren des Lösungsmittels auf übliche Weise trocknet.

2. Verfahren nach Anspruch 1, dadurch gekennzeichnet, daß man die Mischung in flüssigen Stickstoff eintropft.

3. Verfahren nach Anspruch 1 oder 2, dadurch gekennzeichnet, daß man aus der Mischung Tropfen in annähernd gleichmäßiger vorherbestimmter Form mittels eines Dosiersystems herstellt.

4. Verfahren nach einem der Ansprüche 1 - 3, dadurch gekennzeichnet, daß man die Pulver, Granulate oder Pellets gefriertrocknet.

5. Verfahren nach einem der Ansprüche 1 - 4, dadurch gekennzeichnet, daß man die Mischung aus Wirkstoff und Gerüstbildner mit einem zusätzlichen Gerüstbildner, ausgewählt aus der Gruppe bestehend aus:
   Albumine, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylalkchol, Polyvinylpyrrolidon, Polyacrylsäure und Polymere aus Methacrylsäure und Methacrylsäureestern, Celluloseacetatphthalat, Hydroxypropylmethylcellulosephthalat, azo-vernetzte Polymethacrylate, Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden, Galactomannanderivate wie Ethyl- oder Acetylgalactomannane, mit Adipinsäure vernetzte Polysaccharide, lipophile Substanzen wie abbaubare Mono-, Di-, und Triglyceride, erodierbare Fettalkohole; sowie deren Mischungen, versetzt.

6. Verfahren nach einem der Ansprüche 1 - 5, dadurch gekennzeichnet, daß der Mischung aus Gerüstbildner und Wirkstoff Weichmacher und Geschmackskorrigentien, ausgewählt aus der Gruppe bestehend aus:
   Glycerol, Propylenglykol, Polyethylenglykol, Triacetin, Sorbitol, Sorbitangemische, Glucosesirup, und deren Mischungen,
   zugesetzt werden.

7. Verfahren nach einem der Ansprüche 1 - 6, dadurch gekennzeichnet, daß als Gerüstbildner Gelatine mit einem Maximum der Molekulargewichtsverteilung oberhalb $10^5$ D bei maximal 70°C mit dem Wirkstoff vermischt wird.

8. Verfahren nach einem der Ansprüche 1 - 7, dadurch gekennzeichnet, daß der Wirkstoff in gelöster, mikroverkapselter, nanoverkapselter, feindisperser oder in konjugierter Form an dem hydrophilen Makromolekül, vorgelegt wird.

9. Verwendung der Pulver, Granulate oder Pellets, hergestellt nach dem Verfahren nach einem der Ansprüche 1 bis 8 zur Herstellung einer kosmetischen Zubereitung.

10. Verwendung der Pulver, Granulate oder Pellets, hergestellt nach dem Verfahren nach einem der Ansprüche 1 bis 6, zur Herstellung einer Zubereitung, die ausgewählt ist aus der Gruppe bestehend aus:
    pharmazeutischen, diagnostischen, analytischen Zubereitung.

**11.** Verwendung nach Anspruch 10 zur Herstellung einer Zubereitung, die ausgewählt ist aus der Gruppe bestehend aus:
nasalen, bukkalen, oralen sowie peroralen Heilmitteln.

**12.** Pharmazeutische Zubereitung, enthaltend Pulver, Granulate oder Pellets, hergestellt nach dem Verfahren nach einem der Ansprüche 1 bis 8.

**13.** Kosmetische Zubereitung, enthaltend Pulver, Granulate oder Pellets, hergestellt nach dem Verfahren nach einem der Ansprüche 1 bis 8.

**14.** Health care produkt, enthaltend Pulver, Granulate oder Pellets, hergestellt nach dem Verfahren nach einem der Ansprüche 1 bis 8.

**Claims**

**1.** Process for the production of powders, granules or pellets containing at least one water-soluble active ingredient, characterized in that

a) a bulking agent consisting of hydrophilic macromolecules selected from the group consisting of:
collagen, gelatin, fractionated gelatin, collagen hydrolysates, gelatin derivatives, vegetable proteins, vegetable protein hydrolysates, elastin hydrolysates, is dissolved in an aqueous or aqueous/organic solvent,
b) the active ingredient is dissolved or suspended in the solution according to a) and
c) the resulting mixture of dissolved bulking agent and dissolved or suspended active ingredient is added dropwise to an inert liquefied gas at low temperature, and thus powders, granules or pellets are formed, and
d) the powders, granules or pellets formed in this way are dried by evaporation or sublimation of the solvent in a conventional way.

**2.** Process according to Claim 1, characterized in that the mixture is added dropwise to liquid nitrogen.

**3.** Process according to Claim 1 or 2, characterized in that drops are produced in approximately uniform predetermined shape from the mixture by means of a metering system.

**4.** Process according to any of Claims 1-3, characterized in that the powders, granules or pellets are freeze-dried.

**5.** Process according to any of Claims 1-4, characterized in that the mixture of active ingredient and bulking agent is mixed with an additional bulking agent selected from the group consisting of:
albumins, agar-agar, gum arabic, pectins, tragacanth, xanthan, natural and modified starches, dextrans, dextrins, maltodextrin, chitosan, alginates, cellulose derivatives, polyvinyl alcohol, polyvinylpyrrolidone, polyacrylic acid and polymers of methacrylic acid and methacrylic esters, cellulose acetate phthalate, hydroxypropylmethylcellulose phthalate, azo-crosslinked polymethacrylates, polyurethane/sugar copolymers, particularly suitable sugar components being oligomeric galactomannans or galactomannan derivatives, which are then crosslinked with aliphatic diisocyanates, galactomannan derivatives such as ethyl- or acetylgalactomannans, polysaccharides crosslinked with adipic acid, lipophilic substances such as degradable mono-, di- and triglycerides, erodable fatty alcohols; and mixtures thereof.

**6.** Process according to any of Claims 1-5, characterized in that plasticizers and masking flavours selected from the group consisting of:
glycerol, propylene glycol, polyethylene glycol, triacetin, sorbitol, sorbitan mixtures, glucose syrup, and mixtures thereof, are added to the mixture of bulking agent and active ingredient.

**7.** Process according to any of Claims 1-6, characterized in that gelatin with a maximum in the molecular weight distribution above $10^5$ D is mixed as bulking agent with the active ingredient at 70°C maximum.

**8.** Process according to any of Claims 1-7, characterized in that the active ingredient is in dissolved, microencapsulated, nanoencapsulated or microdisperse form or in conjugated form to the hydrophilic macromolecule.

**9.** Use of the powders, granules or pellets produced by the process according to any of Claims 1 to 8 for producing a cosmetic preparation.

**10.** Use of the powders, granules or pellets produced by the process according to any of Claims 1 to 8 for producing a preparation selected from the group consisting of:
pharmaceutical, diagnostic, analytic preparation.

**11.** Use according to Claim 10 for producing a preparation which is selected from the group consisting of:
nasal, buccal, oral and peroral medicines.

**12.** Pharmaceutical preparation containing powders, granules or pellets produced by the process according to any of Claims 1 to 8.

**13.** Cosmetic preparation containing powders, granules or pellets produced by the process according to any of Claims 1 to 8.

14. Health care product containing powders, granules or pellets produced by the process according to any of Claims 1 to 8.

## Revendications

1. Procédé de production de poudres, granules ou pellets contenant au moins une substance active soluble dans l'eau, caractérisé en ce que :

   a) on dissout dans un solvant aqueux ou aqueux-organique une matière de structuration formée de macromolécules hydrophiles choisies dans le groupe consistant en :
   collagène, gélatine, gélatine fractionnée, hydrolysats de collagène, dérivés de gélatine, protéines végétales, hydrolysats de protéines végétales, hydrolysats d'élastine,
   b) on dissout ou met en suspension la substance active dans la solution selon a) et
   c) on fait tomber goutte à goutte dans un gaz inerte liquéfié à basse température le mélange obtenu constitué de matière de structuration dissoute et de substance active en solution ou en suspension et on forme ainsi les poudres, granules ou pellets, et
   d) on sèche de manière classique les poudres, granules et pellets ainsi formés par évaporation ou sublimation du solvant.

2. Procédé suivant la revendication 1, caractérisé en ce qu'on verse le mélange goutte à goutte dans de l'azote liquide.

3. Procédé suivant la revendication 1 ou 2, caractérisé en ce qu'on produit à partir du mélange des gouttes de forme prédéterminée à peu près régulières au moyen d'un système doseur.

4. Procédé suivant l'une des revendications 1 à 3, caractérisé en ce qu'on lyophilise les poudres, les granules ou les pellets.

5. Procédé suivant l'une des revendications 1 à 4, caractérisé en ce qu'on ajoute au mélange de substance active et de matière de structuration une matière de structuration supplémentaire choisie dans le groupe consistant en :
   albumine, agar-agar, gomme arabique, pectine, gomme adragante, xanthane, amidons naturels ainsi que modifiés, dextranes, dextrines, maltodextrine, chitosane, alginates, dérivés de cellulose, polymère d'alcool vinylique, polyvinylpyrrolidone, polymère d'acide acrylique et polymères d'acide méthacrylique et d'esters d'acide méthacrylique, acétate-phtalate de cellulose, phtalate d'hydroxypropylméthylcellulose, polyméthacrylates azo-réticulés, copolymères polyuréthanne-sucre, auquel cas des galactomannanes ou dérivés de galactomannanes oligomères conviennent particulièrement comme composant sucre, qui sont ensuite réticulés avec des diisocyanates aliphatiques, des dérivés de galactomannane tels qu'éthyl- ou acétyl-galactomannanes, des polysaccharides réticulés avec l'acide adipique, des substances lipophiles telles que mono-, di- et triglycérides dégradables, des alcools gras éliminables par érosion ; ainsi que des mélanges de ces matières.

6. Procédé suivant l'une des revendications 1 à 5, caractérisé en ce qu'on ajoute au mélange de matière de structuration et de substance active des plastifiants et des substances corrigeant le goût, choisis dans le groupe consistant en :
   glycérol, propylène-glycol, polyéthylène-glycol, triacétine, sorbitol, mélanges de sorbitanne, sirop de glucose et des mélanges de substances de ce groupe.

7. Procédé suivant l'une des revendications 1 à 6, caractérisé en ce qu'on mélange comme matière de structuration, avec la substance active, de la gélatine ayant un maximum de répartition de poids moléculaire au-dessus de $10^5$ D, à une température maximale de 70°C.

8. Procédé suivant l'une des revendications 1 à 7, caractérisé en ce que la substance active est préparée sous la forme dissoute, micro-encapsulée, nano-encapsulée, finement dispersée ou conjuguée à la macromolécule hydrophile.

9. Utilisation des poudres, granules ou pellets obtenus par le procédé selon l'une des revendications 1 à 8, pour produire une préparation cosmétique.

10. Utilisation des poudres, granules ou pellets obtenus par le procédé selon l'une des revendications 1 à 8, pour produire une préparation qui est choisie dans le groupe consistant en : préparation pharmaceutique, diagnostique, analytique.

11. Utilisation suivant la revendication 10, pour produire une préparation qui est choisie dans le groupe consistant en : médicaments à administrer par voie nasale, buccale, orale ainsi que perorale.

12. Préparation pharmaceutique contenant des poudres, des granules ou des pellets produits par le procédé selon l'une des revendications 1 à 8.

13. Préparation cosmétique, contenant des poudres, granules ou pellets produits par le procédé suivant l'une des revendications 1 à 8.

**14.** Produit de Health care, contenant des poudres, granules ou pellets obtenus par le procédé suivant l'une des revendications 1 à 8.